# EUROPEAN PATENT SPECIFICATION

(11) **EP 3 150 184 B1**
(45) Date of publication and mention of the grant of the patent: **02.05.2018**
(21) Application number: 15187392.4
(22) Date of filing: 29.09.2015
(51) Int. Cl.: A61F 2/46, A61F 2/30

(54) **MEDICAL INSTRUMENT**
MEDIZINISCHES GERÄT
DISPOSITIF MÉDICAL

(43) Date of publication of application: 05.04.2017
(73) Proprietor: Fundacja Rozwoju Kardiochirurgii Im. Prof. Zbigniewa Religi, 41-800 Zabrze (PL)
(72) Inventor: Nawrat, Zbigniew, 41-800 Zabrze (PL); Mucha, Lukasz, 37-700 Przemysl (PL); Lis, Krzysztof, 42-674 Zbroslawice (PL)
(74) Representative: Malcherek, Piotr

(56) References cited:
- US-A- 5 383 900
- US-A1- 2010 136 082
- US-A1- 2013 060 335
- US-B1- 6 213 343
- US-B1- 6 299 018
- US-B2- 8 006 734

## Description

The invention relates to a medical instrument used for implanting medical products and substances into a patient's body. The instrument according to the invention is used in particular for transporting medical products to the place appropriate from a medical point of view in the patient's body. The medical instrument is used, for example, in medical procedures such as: joining tissues, biological regeneration, restoring vitality in damaged parts of the body, stimulating growth of blood vessels, blocking and eliminating cancer cells.

In this description medical product means active biological material, including stem cells, pharmacological materials and substances as well as technical medical devices such as mesh for joining tissues.

### STATE OF THE ART

There is a number of methods and devices known to date enabling transport of cells, for example stem cells, to tissues or transport of such cells with sheets of biological material which constitute "human textile". For example, US2010/0136082 discloses an implantable laminate cartilage repair patch having a first outer cell occlusive layer, a second outer cell porous layer, and a cartilagenic matrix disposed between the first and second layer, including chemical components promoting generation of hyaline-like cartilage in the presence of autologous stem cells. Such cartilage repair patch is implanted intraarticularly and prior to procedure the place of the implant is prepared by applying a layer of fibrin adhesive and a blood clot.

Furthermore, for example US2013/0060335 discloses methods and devices enabling spraying of cell suspension composed of various types of cells onto the surface of the tissue. The sprayed suspension may, for example, contain stem cells in connection with skin cells and some additional biological material. The distribution of the suspension is performed by means of a device which has a needle channel or an appropriate tube provided for this purpose. The suspension in the device assumes the form of droplets dispersed in the air. The device enables appropriate adjustment of the air stream and of the suspension, owing to which it is possible to distribute the suspension in a manner differentiated and suitable for its purpose.

US 6 299 018 discloses a bandage dispensing device with handle portion and head portion. The device is portable and easily accessible due to means for attaching to a person's clothing. The handle portion and the head portion are hollow casings and the head portion is attached at the upper portion of the handle. The handle portion has indentions facilitating a gripping of the device, whereas the head portion has apertures for bandage as well as a removable door for accessing the interior thereof. Means for incrementally advancing bandage are assembled inside the head portion. They comprise a rotatable spool of a bandage assembly strip, a drive belt and means for advancing said drive belt. The strip of bandage assembly generally comprises spaced apart bandages carried by a non-adhering carrying strip having preferably one through hole formed between each bandage, wherein pins perpendicularly extending form the drive belt engage within a plurality of the through holes.

US 8 006 734 discloses an adhesive segment applicator apparatus, which is provided for use with an adhesive segment laden carrier release tape. The adhesive segment applicator apparatus contains a carrier tape dispensing system and an adhesive dispensing wheel. The carrier tape dispensing system includes a spool that is rotatably mounted inside the housing of the adhesive segment applicator apparatus. A roll of adhesive segment laden carrier release tape is inserted onto the supply spool and engages the supply spool in a secure fashion, permitting the roll of adhesive segment laden carrier release tape to rotate when the supply spool rotates. The supply spool has a drive pulley mounted thereon in order to drive a belt. The carrier tape dispensing system also includes a take-up spool that is rotatably mounted inside the housing of the applicator apparatus. The adhesive dispensing wheel is positioned at the nose portion of the adhesive segment applicator apparatus and rotatably mounted external to the housing of the applicator apparatus. This dispensing wheel is used for application of adhesive segments.

### THE AIM OF THE INVENTION

The aim of the invention is to provide a medical instrument which allows implanting into the body the pre-prepared medical products in a specified form. The construction of the instrument should also enable to apply specified substances, for example substances promoting the treatment immediately prior to introducing medical products into the body. Another aim of the invention is to ensure simple and modular construction of the instrument, which should decrease the number of elements which could damage the implanted medical products and to ensure easy sterilisation of certain elements of the instrument. Still another aim of the invention is to ensure that the instrument of the invention is universally applicable, which means that said instrument allows to implant into the body medical products of various type and any dimension. At the same time it is the aim of the invention to provide an instrument of suitable mechanical and physical-chemical parameters promoting integration and further development of the implanted medical product with the body tissues.

### THE ESSENCE OF THE INVENTION

The present invention relates to a medical instrument comprising a body with an outlet opening through which medical products are implanted. The essence of the invention is that inside the body there is a belt for conveying medical products running between at least two rollers, wherein one of said rollers is a guiding roller and the other is a driving roller. Furthermore, the body has an inlet opening for introducing medical products into said body. A route of the belt goes through the inlet opening and subsequently the outlet opening, wherein between the inlet opening and the outlet opening inside the body there is a pressure roller assembly for pressing down medical products conveyed on the belt to a surface of said belt and at least one nozzle for applying auxiliary substances to transported medical product, wherein said nozzle is connected to a respective storage unit. The invention is defined by the appended claim 1.

In the area of the outlet opening on the outer part of the body there is a sliding spring element with a pressure spline for implanting medical products.

It is preferable that there be fasteners for detachable mounting of the container with medical products on the outer part of the housing in the area of the inlet opening.

It is advantageous that the belt have a multitude of splines for holding medical products on said belt.

It is also preferable that the sliding spring element have a fixing base positively shaped to a guide rail inside the body and located by the outlet opening. At the same time the sliding spring element has a scraper mechanism and the belt has at least one longitudinal groove for accommodating scraper elements of the scraper mechanism.

It is also preferable that the storage unit be equipped with a drive.

It is particularly advantageous that the driving roller be driven with a motor coupled therewith by means of a belt transmission.

### ADVANTAGES OF THE INVENTION

The main advantage of the invention is ensuring the possibility to implant into the body medical products pre-prepared in a specified form. Moreover, the construction of the instrument enables the application of certain substances promoting the treatment immediately prior to introducing medical products into the body, which is another advantage of the invention. The construction of the instrument ensures a system of safe transport and implanting the medical products, where the transported medical products are not prone to damage or destruction. Moreover, in the last stage prior to implanting medical products it is possible to deliver the appropriately selected auxiliary substances such as chemical, biological or physical agents, which results in the possibility to create various structures from the implanted medical products within the patient's body and enables to introduce auxiliary substances simultaneously with the medicinal substances, wherein said auxiliary substances influence not only the provided medicinal substances but also the whole of the patient's body. Still another advantage of the instrument is the possibility to implant medical products of various characteristics, owing to which heterogeneous layered structures may be created in the patient's body. The present disclosure in particular allows to implant medical products in the form of bio-absorbable matrices containing stem cells, in which case the advantages of the invention, such as avoiding damage to the transported material and the possibility to deliver auxiliary substances directly onto the transported medical product, are evident. Implanting the medical products with the use of the instrument according to the invention ensures maintaining the appropriate mechanical and physical-chemical parameters and enables the creation of conditions promoting integration of the implanted medicinal substance with the body tissues and further development thereof. Furthermore, a simple and uncomplicated construction of the medical instrument enables quick adjustment thereof to various implanted medical products as well as quick sterilisation of certain components of the instrument.

### DESCRIPTION OF THE DRAWINGS

The invention is presented in more detail in the embodiments and in the drawing, where:

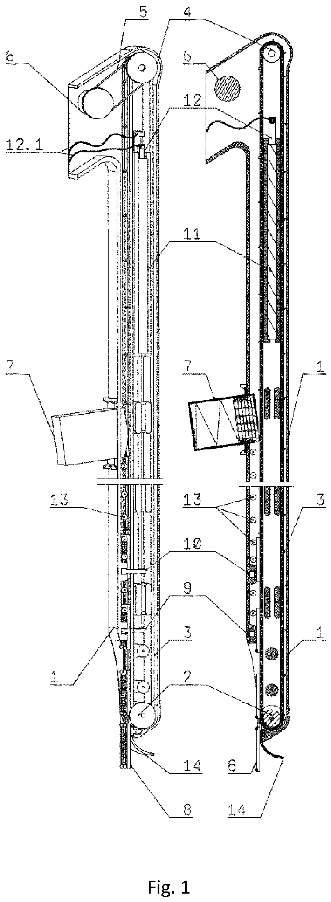

Fig. 1 is a perspective side view of the inside of the medical instrument in connection with a longitudinal sectional view of the instrument,

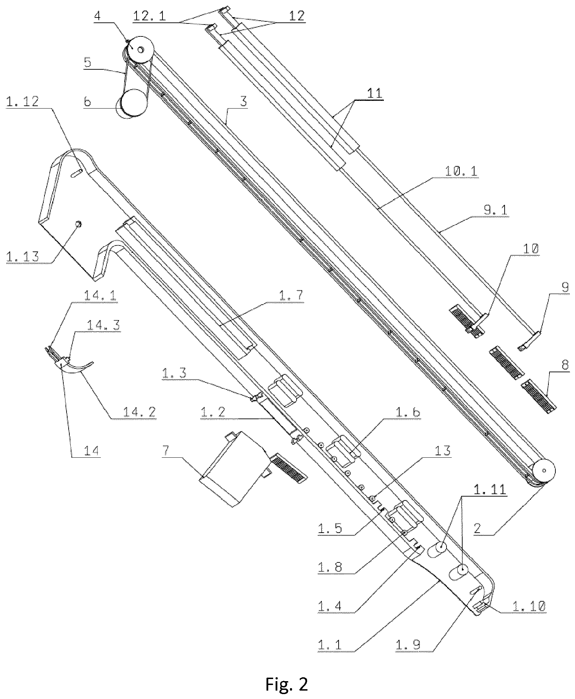

Fig. 2 is a perspective exploded view of the medical instrument,

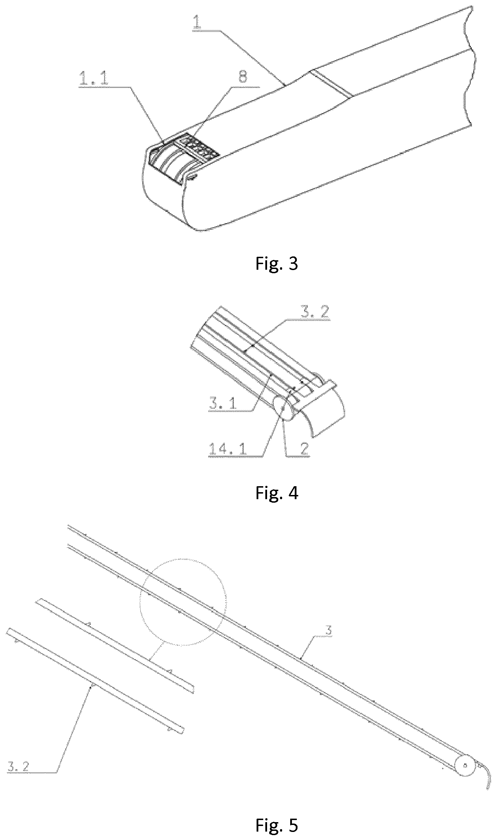

Fig. 3 is a simplified view of the body of the medical instrument,
Fig. 4 is a belt and a sliding spring element,
Fig. 5 is a side perspective view of the belt and a detailed side view of a section of the belt,

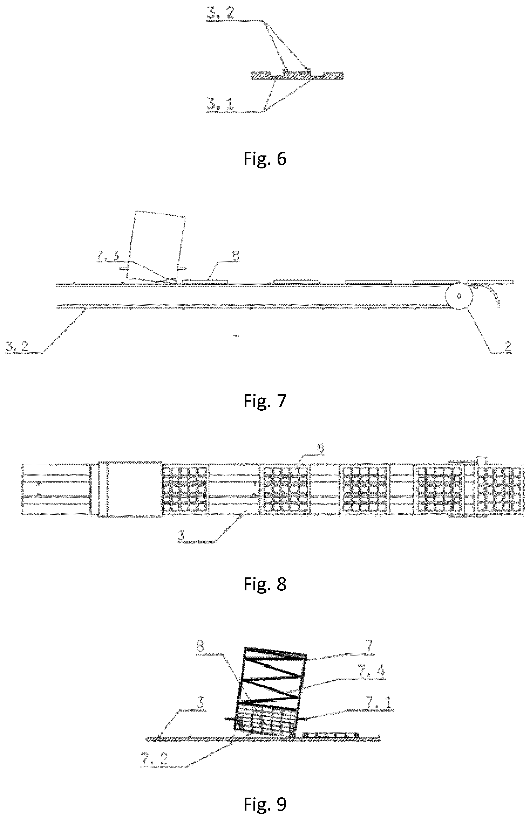

Fig. 6 is a cross-sectional view of the belt,
Fig. 7 is a side view of the belt with the guiding roller and the sliding spring element,
Fig.8 is a top view of the belt,
Fig. 9 is the belt with an exemplary container with medical products,
Fig. 10 is a perspective view of the exemplary container with medical products,
Fig. 11 is a schematic view of the medical instrument where a belt of limited length was applied.

### EMBODIMENT

The medical instrument has been presented in a detailed embodiment, where a belt 3 in the form of a continuous loop was applied.

The medical instrument comprises a body 1 inside which there is an inlet opening 1.2 for introducing medical products thereto and an outlet opening 1.1 through which medical products are implanted. On the outer side of the body 1 in the area of the inlet opening there are fasteners 1.3 for detachable mounting a container 7 with medical products. The body 1 is made of two parts fixed together by means of screws going through holes 1.11.

In the presented embodiment the container 7 with medical products is connected to the fasteners 1.3. On the outside the container has two fixing splines 7.1 cooperating with the fasteners 1.3. The medical products in the container 7 are provided in the form of three-dimensional matrices 8 containing active biological material - stem cells. In the lower part of the container, going into the inlet opening 1.2 there is a sliding base 7.2 and an outlet window 7.3 for matrices. Inside the container there is a spring 7.4 for pressing down the matrices 8 in the direction of the outlet window 7.3. It is evident that in the inlet opening 1.2 a different kind of the container 7 with medical products may be mounted. The medical product itself may also have a different form, volume and application. Through the inlet opening 1.2 into the body 1 active biological material may be introduced, wherein said material may contain stem cells, it may be pharmacological material for treating inflammation or medical technical material as, for example, mesh for joining tissues.

Inside the body there is a continuous belt 3 for conveying medical products in the form of matrices 8. The belt 3 goes between two rollers 2, 4, wherein the first of said rollers is a guiding roller 2 and the second of said rollers is a driving roller 4. The guiding roller 2 is mounted on a bolt 1.9 constituting a part of the body 1. The driving roller 4 is coupled with a motor 6 by means of a belt transmission 5. The driving roller 4 is mounted on a bolt 1.12 constituting a part of the body 1. The motor is mounted in the body 1 with an auxiliary, not shown in the drawing, fixing element going through a through hole 1.13 made in the body 1. The application of the belt transmission 5 enables an advantageous spatial location of the motor 6 and of the driving roller 4 without increasing the size of the instrument. It is also possible to mount the driving roller 4 directly on the drive shaft of the motor 6 or coupling the driving roller 4 with the motor 6 by means of a different, selected transmission.

The continuous belt 3 has on the surface thereof two longitudinal grooves 3.1 and is equipped with a multitude of splines 3.2 for pulling matrices 8 out of the outlet window 7.3 of the container 7 and preventing sliding of the matrices 8 on the belt 3 and ensuring their constant position on the belt 3. Other embodiments of the belt are also possible, for example, with a smooth surface of the belt 3 covered with a substance allowing to temporarily attach the matrix 8 to the belt 3 and withdrawing it from the container 7 and subsequently moving said matrix to the outlet opening 1.1.

The route of the belt 3 inside the body 1 goes through the inlet opening 1.2 and subsequently through the outlet opening 1.1. Between said openings 1.2 and 1.1 inside the body 1 there is a pressure roller assembly 13 pressing down to the surface of the belt 3 the matrices 8 conveyed on said belt 3 and two dosing nozzles 9, 10 for applying auxiliary substances to the transported medical product. Single rollers of the roller assembly 13 are mounted on bolts 1.8 constituting a part of the body 1. Both nozzles 9, 10 are mounted in splines 1.4, 1.5 of the body 1 and are connected by means of tubes 9.1, 10.1 with respective storage units 11 containing substances for promoting the treatment process. The tubes 9.1, 10. 1 are located between internal supports 1.6 of the body 1. The storage units 11 are also mounted in different internal supports 1.7 of the body 1. For example, inside the storage unit 11 there may be adhesive for joining the matrix 8 with the surface of the organ onto which said matrix 8 is to be laid and also preventing further fragmentation of tissues in the patient's body. In the storage unit 11 growth hormones may also be inserted or nutritional substances. Adding the specified substances into the medical product, directly prior to implantation thereof into the patient's body prevents drying and enables to determine the fixation time of active compounds and mutual interaction of the medical product and the applied substances. The storage units 11 are cylindrical in shape and have a piston, not presented in detail, coupled with a linear drive 12. Control of the drive 12 results in the piston operating inside the storage unit 11 and adding the auxiliary substances into the matrices 8 by means of the nozzles 9, 10.

In the area of the outlet opening 1.1 there is a sliding spring element 14 with a pressure spline 14.2 for implanting medical products - the matrices 8. The fixing base 14.3 of the sliding spring element 14 is positively shaped with regard to the to the guide rail 1.10 located inside the body 1 by the outlet opening 1.1. The fixing base 14.3 is releasably connected to the guide rail 1.10. At the same time the sliding spring element is equipped with a scraper mechanism 14.1, the scraper elements of which go into grooves 3.1 of the belt 3, thus removing the transported matrices 8 from the belt 3. If the scraper mechanism 14.1 is not present, the matrix 8 slides down along the sliding spring element 14.

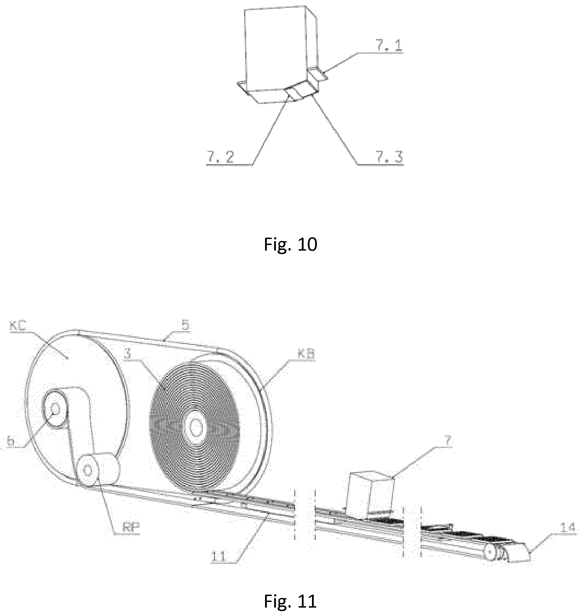

It is also possible to provide a medical instrument with a belt 3 in the form of a limited-length belt (fig. 11). In this embodiment a driven wheel KB with a wound belt 3 and a driving wheel KC onto which the belt 3 is wound are mounted inside the body. The active driving wheel KC is an equivalent of the driving roller 4 and is mounted directly on the motor 6 shaft. The driving wheel KC and the driven wheel KB are connected by means of a belt transmission 5. In the area of the driving wheel KC there is an auxiliary roller RP guiding the belt 3 while the instrument is at work, that is while the belt 3 is being wound from the driven wheel KB onto the driving wheel KC.

In each of the embodiments it is possible to mount the body 1 of the medical instrument from the side of the drive 6 on a surgical robot or to connect said body 1 to a handle enabling manual operation of the instrument.

While the medical instrument is at work the driven belt 3 moving inside the body 1 removes the matrices 8 from the outlet window 7.3 of the container 7 by means of splines 3.2 and conveys said matrices 8 from the inlet opening 1.2 to the outlet opening 1.1. While moving inside the instrument the matrices 8 are pressed down by a pressure roller assembly 13 to the surface of the belt 3, and subsequently said matrices 8 are moved to the area of the nozzles 9, 10 by means of which the desired, auxiliary substances may be applied onto the matrices 8. Application of both substances is executed from the storage units 11 and is conducted as a result of operating the drives 12 controlled by means of a cable terminal 12.1. After the application of the auxiliary substances onto the matrices 8 they are removed from the belt 3 by means of the scraper elements 14.1 running in the grooves 3.1 of the belt 3. Therefore the matrices 8 go out of the body 1 through the outlet opening 1.1 and subsequently the matrices 8 are attached to an organ in the patient's body and pressed thereto with a pressure spline 14.2.

## Claims

1. A medical instrument comprising a body (1) with an outlet opening (1.1) for implanting medical products, wherein inside the body (1) there is a belt (3) for conveying medical products running between at least two rollers (2, 4), wherein one of said rollers is a guiding roller (2) and the other is a driving roller (4), moreover the body (1) has an inlet opening (1.2) provided for introducing medical products into the body (1), and a route of the belt (3) goes through the inlet opening (1.2) and subsequently the outlet opening (1.1), wherein between the inlet opening (1.2) and the outlet opening (1.1) inside the body (1) there is a pressure roller assembly (13) provided for pressing down medical products conveyed on the belt (3) to a surface thereof and at least one nozzle (9) for applying auxiliary substances to transported medical product, wherein said nozzle (9) is connected to a respective storage unit (11), and in the area of the outlet opening (1.1) on the outer surface of the body (1) there is a sliding spring element (14) with a pressure spline (14.2) provided for implanting medical products.

2. The instrument according to claim 1, **characterised in that** on the outer surface of the body (1) in the area of the inlet opening (1.2) there are fasteners (1.3) for releasable mounting of a container (7) with medical products.

3. The instrument according to claim 1 or 2, **characterised in that** the belt (3) has a multitude of splines (3.2) provided for holding medical products on the surface of the belt (3).

4. The instrument according to one of the claims 1-3, **characterised in that** the sliding spring element (14) has a fixing base (14.3) positively shaped in relation to a guide rail (1.10) inside the body (1) and located by the outlet opening (1.1) and is equipped with a scraper mechanism (14.1) and the belt (3) has at least one longitudinal groove (3.1) into which enter scraper elements of the scraper mechanism (14.1).

5. The instrument according to claims 1-4, **characterised in that** the storage unit (11) is equipped with a drive (12).

6. The instrument according to claims 1-5, **characterised in that** the driving roller (4) is driven with a motor (6) coupled therewith by means of a belt transmission (5).

## Patentansprüche

1. Medizinisches Werkzeug mit einem Körper (1) mit einer Austrittsöffnung (1.1) zum Implantieren von medizinischen Produkten, **dadurch gekennzeichnet, dass** sich im Inneren des Körpers (1) ein Band (3) für Verlagerung von medizinischen Produkten befindet, das zwischen mindestens zwei Rollen (2, 4) durchläuft, wobei eine der Rollen eine Führungsrolle (2) und eine andere eine Antriebsrolle (4) ist, darüber hinaus der Körper (1) eine Einlassöffnung (1.2) zum Einbringen von medizinischen Produkten in den Körper (1) aufweist, und das Band (3) nacheinander durch die Einlassöffnung (1.2) und durch die Austrittsöffnung (1.1) durchläuft, wobei innerhalb des Körpers (1) zwischen der Einlassöffnung (1.2) und der Austrittsöffnung (1.1) eine Druckrollenanordnung (13), um die auf dem Band (3) geförderten medizinischen Produkte auf dessen Oberfläche zu drücken, sowie mindestens eine Düse (9) zum Auftragen von Hilfsstoffen auf die transportierten medizinischen Produkte vorgesehen sind, wobei die Düse (9) mit einem zugeordneten Vorratsbehälter (11) verbunden ist und im Bereich der Austrittsöffnung (1.1) an der Außenseite des Körpers (1) ein Gleitfederelement (14) mit einer Klemmzunge (14.2) zur Implantation von medizinischen Produkten lokalisiert ist.

2. Medizinisches Werkzeug nach Anspruch 1, **dadurch gekennzeichnet, dass** sich auf der Außenseite des Körpers (1) im Bereich der Einlassöffnung (1.2) Verriegelungen (1.3) zur lösbaren Befestigung des Behälters (7) mit medizinischen Produkten befinden.

3. Medizinisches Werkzeug nach Anspruch 1 oder 2, **dadurch gekennzeichnet, dass** das Band (3) eine Vielzahl von Keilnuten (3.2) aufweist, die zum Halten von medizinischen Produkten auf der Oberfläche des Bandes (3) bestimmt sind.

4. Medizinisches Werkzeug nach einem der Ansprüche 1 - 3, **dadurch gekennzeichnet, dass** das Gleitfederelement (14) eine zu einer Führungsschiene (1.10) formschlüssig geformte und im Inneren des Körpers (1) an der Austrittsöffnung (1.1) angeordnete Fixierbasis (14.3) aufweist und mit einem Abstreifmechanismus (14.1) ausgestattet ist, und das Band (3) mindestens eine Längsnut (3.1) aufweist, in die die Abstreifelemente des Abstreifmechanismus (14.1) eingreifen.

5. Medizinisches Werkzeug nach den Ansprüchen 1 - 4, **dadurch gekennzeichnet, dass** der Vorratsbehälter (11) mit einem Antrieb (12) ausgestattet ist.

6. Medizinisches Werkzeug nach den Ansprüchen 1 - 5, **dadurch gekennzeichnet, dass** die Antriebsrolle (4) mit einem damit gekoppelten Motor (6) mittels eines Riemengetriebes (5) angetrieben

## Revendications

1. Dispositif médical possédant un corpus (1) muni d'une orifice de sortie (1.1) à implanter les matériaux thérapeutiques **caractérisé en ce qu'**à l'intérieur du corpus (1) est inséré une bande (3) à déplacer les matériaux thérapeutiques qui passe entre au moins deux rouleaux (2, 4), où l'un des rouleaux est un rouleau de guidage (2) et le deuxième est un rouleau entraîneur (4), en plus le corpus (1) possède une orifice d'entrée (1.2) à introduire les matériaux thérapeutiques à l'intérieur du corpus (1), et le parcours de la bande (3) couvre successivement l'orifice d'entrée (1.2) et l'orifice de sortie (1.1), où, entre l'orifice d'entrée (1.2) et l'orifice de sortie (1.1), à l'intérieur du corpus (1), est inséré un groupe des rouleaux de pression (13) servant à presser par le dessus les matériaux thérapeutiques transportés par la bande (3) contre la surface de cette bande (3), et, au moins, une tuyère (9) à doser des substances supplémentaires au matériel thérapeutique transporté, reliée avec un réservoir de stockage lui affecté, et, dans la zone de l'orifice de sortie (1), sur la partie extérieure du corpus, est situé un élément élastico - glissant (14) avec une busette de pression (14.2) destinée à implanter les matériaux thérapeutiques.

2. Dispositif selon la revendication 1, **caractérisé en ce que** les verrous (1.3) permettant une fixation séparable du réservoir (7) avec les matériaux thérapeutiques sont situés sur la partie extérieure du corpus (1), dans la zone de l'orifice d'entrée (1.2).

3. Dispositif selon les revendications 1 ou 2, **caractérisé en ce que** la bande (3) possède plusieurs renflements (3.2) destinés à maintenir les substances thérapeutiques sur la surface de la bande (3).

4. Dispositif selon l'une des revendications 1 - 3, **caractérisé en ce que** l'élément élastico -glissant (14) possède une base de fixation (14.3) dont la forme est ajustée à la glissière (1.10) insérée à l'intérieur du corpus (1) et située à côté de l'orifice de sortie (1.1), et, il est équipé d'un mécanisme de raclage (14.1), et, en même temps, la bande (3) possède au moins une gorge longitudinale (3.1) où entrent les éléments racleurs du mécanisme de raclage (14.1).

5. Dispositif selon les revendications 1 - 4, **caractérisé en ce que** le réservoir de stockage (11) est équipé d'une source d'entraînement (12).

6. Dispositif selon les revendications 1 - 5, **caractérisé en ce que** le rouleau entraîneur (4) est propulsé par un moteur (6) auquel il est accouplé à l'aide d'une transmission à courroie (5).
